# EUROPEAN PATENT APPLICATION

(11) **EP 2 896 378 A1**
(43) Date of publication of application: **22.07.2015**
(21) Application number: 15151679.6
(22) Date of filing: 19.01.2015
(51) Int. Cl.: A61B 17/70

(54) **Quick-lock driver for a bone screw**

(30) Priority: 20.01.2014 US 201414158940
(71) Applicant: Zimmer Spine, Inc., Minneapolis, MN 55439 (US)
(72) Inventor: Lemoine, Jeremy J., Minneapolis, MN Minnesota 55439 (US); Vedula, Krishna C., Minneapolis, MN Minnesota 55439 (US); Dickson, Andrew M., Minneapolis, MN Minnesota 55439 (US)
(74) Representative: Mays, Julie

(57) **Abstract**

A driver instrument for engaging and transferring rotational torque to a bone screw assembly which includes a shaft and sleeve adapted to engage and couple the bone screw assembly into a single coaxial unit while the bone screw assembly is being driven into a bone and then to release the bone screw assembly from the driver instrument. The driver instrument includes an elongate shaft, a sleeve, and bone screw assembly engaging features on the elongate shaft and/or the sleeve.

## Description

### TECHNICAL FIELD

The disclosure is directed to a driver instrument for screwing a bone screw into a bony structure. More particularly, the disclosure is directed to a driver instrument configured to facilitate quick alignment and engagement between a bone screw assembly and a driver instrument advanced into the driver socket of the bone screw.

### BACKGROUND

Bone anchors, such as bone screws, are commonly used in surgical procedures to attach constructs to a bony structure of a patient. For example, spinal stabilization constructs configured to stabilize a spinal segment may utilize bone screws to secure spinal rods or other elongate members to one or more vertebrae of a spinal column by means of a housing fixed to the vertebrae by the bone screw. The bone anchors may be screwed into the bony structure with a driver instrument.

It is desirable to quickly secure the housing and bone screw of a bone screw assembly to a driver instrument such that the screw extends generally coaxially from the driver instrument and is retained coupled to the distal end of the driver instrument during initial engagement of the screw with the bony structure; during advancement of the screw into the bony structure; and may be quickly decoupled from the distal end of the driver instrument following final positioning of the bone screw.

### SUMMARY

In one embodiment, this disclosure relates to a driver instrument adapted to engage and drive bone screw assemblies which include a bone screw and an associated housing, said driver instrument comprising an elongate shaft having a proximal end and a distal end, the distal end further comprising a driver engagement feature adapted to engage and rotationally drive a head of a bone screw and one or more lugs protruding radially therefrom, said one or more lugs being adapted to engage a housing of a bone screw assembly to limit axial rotation thereof relative to said elongate shaft; and a sleeve having a proximal end, a distal end, and being adapted for coaxial rotation about a common longitudinal axis with the elongate shaft, wherein the distal end of the sleeve includes an engagement feature adapted to rotationally engage a mating engagement feature of the housing of a bone screw assembly.

In another embodiment, this disclosure relates to a method of engaging and a driver instrument with a bone screw assembly comprising:
inserting a distal tip of a driver instrument having an elongate shaft including a driver engagement feature into a driver socket of a bone screw of a bone screw assembly;
inserting one or more lugs of the elongate shaft into mating channels of a housing of the bone screw assembly, wherein inserting one or more lugs of the elongate shaft into mating channels of the housing of the bone screw assembly limits rotation of the housing relative to the elongate shaft;
inserting a sleeve coaxially disposed about the elongate shaft into the housing of the bone screw assembly with the sleeve in a first position, said sleeve including an engagement feature being adapted to rotationally engage a mating engagement feature of the housing of the bone screw assembly; and
rotating the sleeve relative to the elongate shaft and the housing of the bone screw assembly to a second position while positioned in the housing of the bone screw assembly, wherein upon relative rotation of the sleeve with respect to the elongate shaft, the engagement feature of the sleeve couples with the mating engagement feature of the housing of the bone screw assembly.

The above summary of some example embodiments is not intended to describe each disclosed embodiment or every implementation of the aspects of the disclosure.

### BRIEF DESCRIPTION OF THE DRAWINGS

The aspects of the disclosure may be more completely understood in consideration of the following detailed description of various embodiments in connection with the accompanying drawings, in which:
FIG. 1 is a perspective view of an embodiment of an exemplary driver instrument for engaging and rotationally driving a bone screw;
FIG. 2 is a perspective view of the distal region of the driver instrument for engaging and rotationally driving a bone screw of FIG. 1;
FIG. 2A is a perspective view of the distal region of an alternate embodiment of a driver instrument for engaging and rotationally driving a bone screw;
FIG. 3 illustrates optional features of one embodiment of a distal tip of an elongate shaft of an embodiment of a driver instrument for engaging and rotationally driving a bone screw;
FIG. 4 is an end view of the distal tip of the embodiment of FIG. 3;
FIG. 5 is a schematic view of a bone anchor installed in a bony structure by an embodiment of an exemplary driver instrument;
FIG. 6 is a cross-sectional view of a bone anchor suitable for use with an exemplary driver instrument;
FIG. 7A illustrates details of the interactions among the distal end features of an embodiment of an elongate shaft and the distal end of a sleeve of an exemplary driver instrument with a housing of a bone anchor assembly;
FIG. 7B illustrates further details of the interactions among the distal end features of an embodiment of an elongate shaft and the distal end of a sleeve of an exemplary driver instrument with a housing of a bone anchor assembly;
FIG. 7C is a cross-sectional view taken along line 7C-7C of FIG. 7A;
FIG. 8A illustrates details of the interactions among the distal end features of an alternate embodiment of an elongate shaft and the distal end of a sleeve of a driver instrument with a housing of a bone anchor assembly;
FIG. 8B illustrates further details of the interactions among the distal end features of an alternate embodiment of an elongate shaft and the distal end of a sleeve of a driver instrument with a housing of a bone anchor assembly;
FIG. 8C is a cross-sectional view taken along line 8C-8C of FIG. 8A;
FIG. 9 is a partial cutaway view of a handle illustrating one embodiment of a button adapted to control relative rotary motion between an elongate shaft and a sleeve of an illustrative driver instrument;
FIG. 10 illustrates details of the interactions between the button and elongate shaft of FIG. 9 with surrounding structure omitted.
FIG. 11 illustrates further details of the interactions between the button and elongate shaft of FIG. 9 with surrounding structure omitted.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the disclosure.

### DETAILED DESCRIPTION

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many embodiments, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions, ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended only as exemplary. Selected features of any illustrative embodiment may be incorporated into an additional embodiment unless clearly stated to the contrary. It is noted that references in the specification to "an embodiment", "some embodiments", "other embodiments", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect such feature, structure, or characteristic in connection with other embodiments, whether or not explicitly described, unless clearly stated to the contrary.

A bone screw assembly, as described herein, may be a polyaxial, monoaxial or uniplanar bone screw, for example, comprising a threaded shank body having a head portion (e.g., a partial spherical head) and a driver socket, the head portion may be pivotably positioned in a housing, the housing defining an open channel and having a base with a seating surface partially defining a cavity, said housing further comprising two leg portions together defining generally U-shaped channels on opposing sides of the housing adapted to receive and retain an elongate stabilization member. Many such bone screw assemblies include a threaded region within the open channel which extends along the inner surface of the legs of the housing, said threaded region adapted to receive a screw which serves to secure the elongate stabilization member within the two generally U-shaped channels. Other bone screw assemblies include an engagement region configured to engage with another configuration of a locking member inserted between the leg portions to secure an elongate stabilization member therein. The partial spherical surface of the head portion and the seating surface may cooperate to allow rotation and a degree of angulation between the housing and the bone screw. It will be appreciated that a number of variations of bone screw assemblies are known and many such assemblies include retainer structures which hold the bone screw and the housing together as an assembly. Bone screw assemblies suitable for use with the instant driver instruments may include one or both of a protrusion and a groove associated with the legs of the housing which provides a mating engagement feature for an engagement feature of the sleeve of the driver instruments as will be described herein. In some embodiments, the housing may include a plurality of projections or a plurality of grooves which provide the mating engagement feature. In other embodiments, the driver instruments may be adapted to engage a threaded region within the open channel of the housing rather than a separate feature of the housing. In describing the engagement feature of the instant driver instruments and the mating engagement feature of the bone screw assemblies for use therewith, the singular form "feature" will be used as a collective term for all projections, grooves, and combinations thereof which serve to provide an engagement between the sleeve of the driver instrument and the housing of the bone screw as described in detail herein.

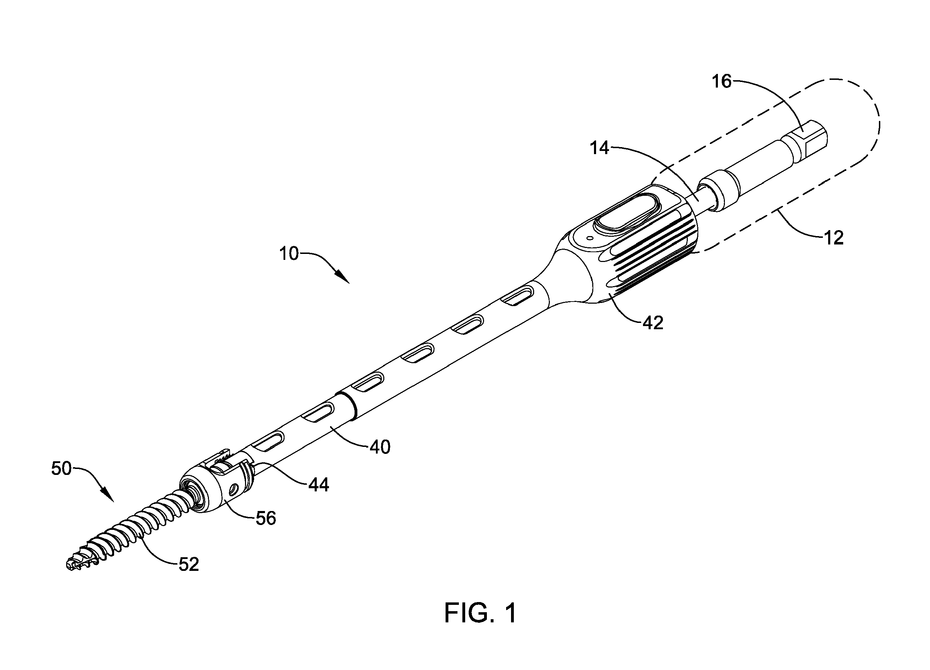

An exemplary driver instrument 10 is illustrated at FIG. 1. The driver instrument 10 includes an optional handle 12 (shown in phantom) and an elongate shaft 14 extending distally from the proximal end 16 of the elongate shaft 14. Surrounding at least a portion of elongate shaft 14 is a sleeve 40 having a sleeve handle 42 at a proximal end thereof and one or more elements of engagement feature 44 proximate a distal end of the sleeve 40.

In some embodiments, the handle 12 may be removably coupled to a proximal end 16 of the elongate shaft 14, such as with a quick-connect feature such as a ball detent. In such embodiments, the quick connect feature optionally may engage a motor-driven element (not shown) associated with the handle 12. In other embodiments, the handle 12 may be permanently coupled to the proximal end 16 of the elongate shaft 14.

As illustrated in FIG. 1, the driver instrument 10 has engaged and is coupled to a bone anchor assembly 50 comprising a bone screw 52 and housing 56.

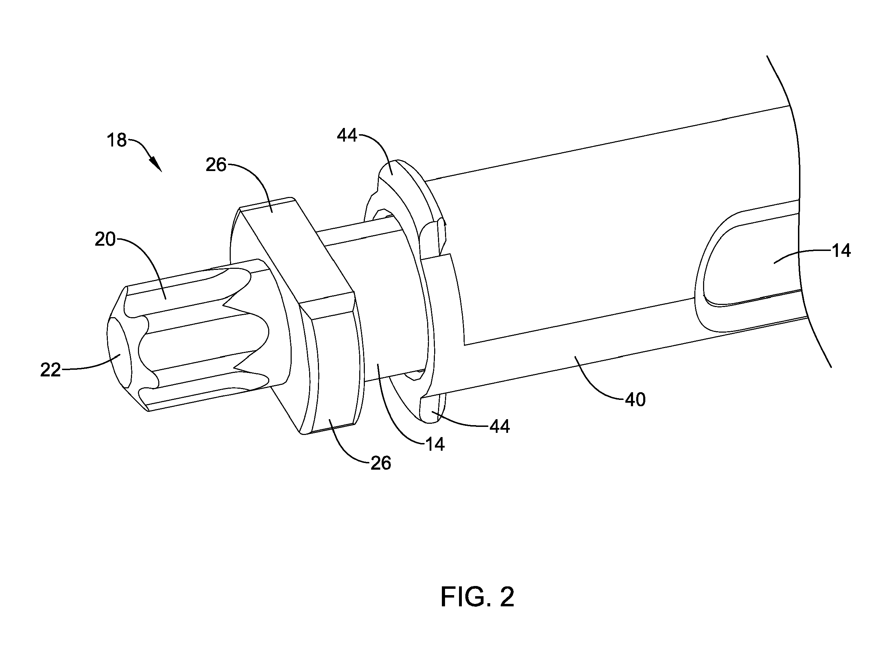

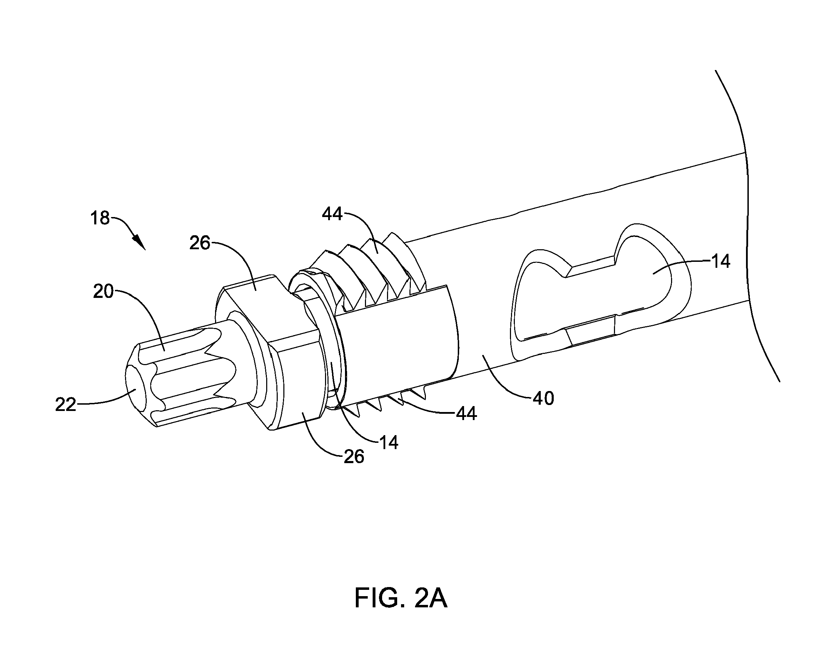

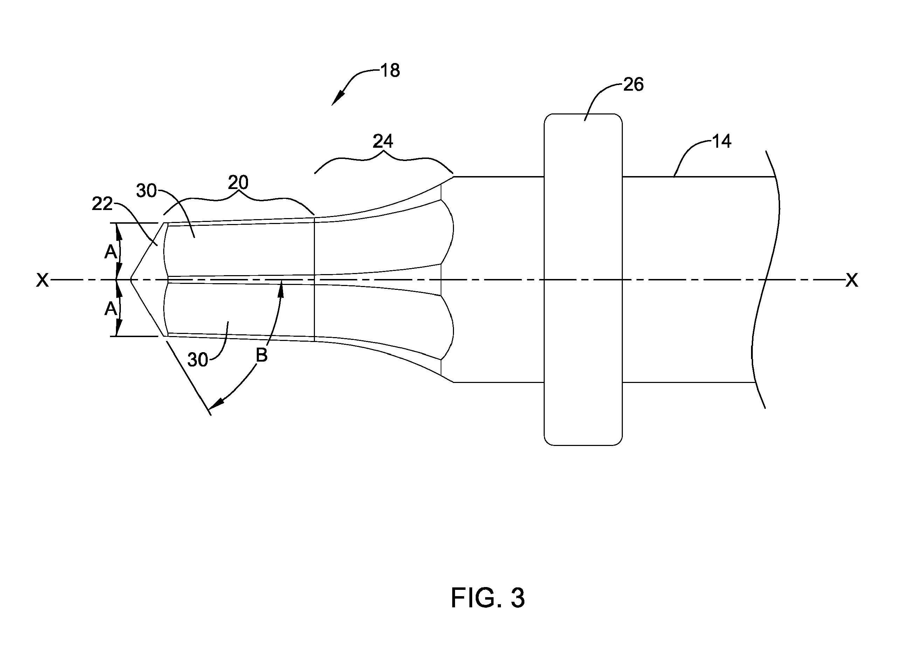

Exemplary aspects of the distal end 18 of the elongate shaft 14 and sleeve 40 are further illustrated in FIGS. 2 and 2A. The distal end 18 of the elongate shaft 14 may include a driver engagement feature 20 and a distal tip 22 extending distally of the driver engagement feature 20 to a distal extremity of the elongate shaft 14. The driver engagement feature 20 may be of any desired configuration for mating with a complementary driver engagement feature (e.g., a driver socket 53) formed in a head 54 of a bone screw 52 (both discussed later herein). For example, in some embodiments the driver engagement feature 20 may be a Torx® driver adapted to be inserted into and to operably mate with a driver socket 53 of a bone screw 52. In other embodiments, the driver engagement feature 20 may be a hex driver, such as a hexalobular internal driving feature having six engagement surfaces 30 configured to engage a plurality of mating surfaces of the driver socket 53 of a bone screw head 54, as shown in further detail in FIG. 3. In other instances, the driver engagement feature 20 may have another configuration having any desired number of engagement surfaces and/or features for mating with a driver socket. The elongate shaft 14 may include a transition region 24 proximal of the driver engagement feature 20 creating a transition from the driver engagement feature 20 to the main body portion of the elongate shaft 14 extending to the handle 12, if present.

Referring to FIG. 3, in some embodiments each of the plurality of engagement surfaces 30 of the driver engagement feature 20 may be arranged nonparallel to the longitudinal rotational axis X of the driver instrument 10. For example, each of the plurality of engagement surfaces 30 may be positioned at an angle A from the longitudinal rotational axis, X. In some embodiments, the angle A may be in the range of about 2 to about 8 degrees, about 2 to about 6 degrees, or about 4 to about 6 degrees from the longitudinal axis X, such as about 2 degrees, about 3 degrees, about 4 degrees, about 5 degrees, about 6 degrees, about 7 degrees, or about 8 degrees from the longitudinal rotational axis X. The tapered engagement surfaces 30 of the driver engagement feature 20 may facilitate advancing the driver engagement feature 20 into a mating driver socket 53, for example.

Furthermore, the distal tip 22 may be a tapered distal tip extending from the driver engagement feature 20 to the distal extremity of the driver instrument 10. In some embodiments, the tapered distal tip 22 may be tapered at an angle B from the longitudinal rotational axis X. In some embodiments, the angle B may be in the range of about 25 degrees to about 75 degrees, about 30 degrees to about 60 degrees, or about 45 degrees to about 60 degrees, such as about 30 degrees, about 45 degrees or about 60 degrees.

Accordingly, the second angle B of the tapered distal tip 22 may be greater than the first angle A between the engagement surfaces 30 and the longitudinal rotational axis X. Thus, the driver engagement feature 20 may be tapered toward the distal tip 22 a first amount, and the distal tip 22 may be tapered toward the distal extremity of the elongate shaft 14 a second amount greater than the first amount.

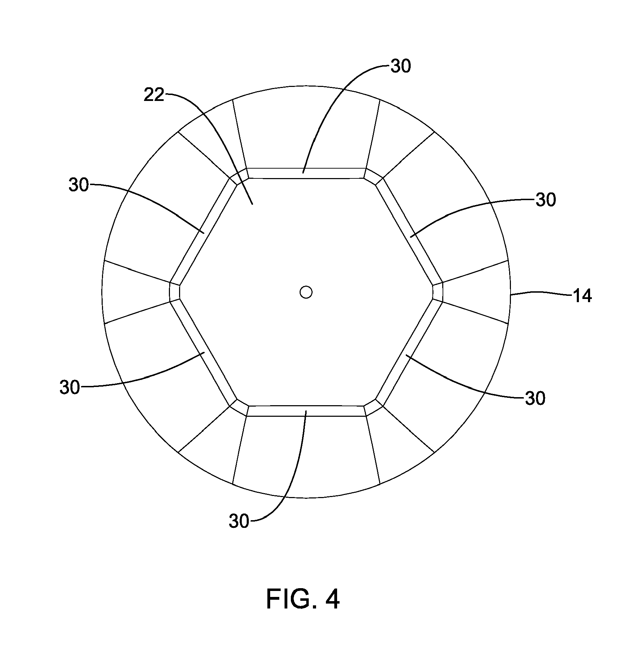

FIG. 4 is an end view of the driver instrument 10 showing six engagement surfaces 30 of a driver engagement feature 20 uniformly arranged in a polygonal configuration centered on the longitudinal rotational axis of the elongate shaft 14 of the driver instrument 10.

Returning to FIG. 2, the elongate shaft 14 may include one or more, or a plurality of lugs 26 located proximal of the driver engagement feature 20, configured to be positionable in the channels 57 of the housing 56 to limit rotation therebetween. As shown in FIG. 2, the elongate shaft 14 may include first and second lugs 26 extending in opposite directions from the elongate shaft 14, for instance. The distal end of the sleeve 40 may be positioned proximal of the lugs 26, with the elongate shaft 14 extending through the lumen of the sleeve 40 such that the distal end 18 of the elongate shaft 14 extends distal of the sleeve 40. The sleeve 40 may be rotatable relative to the elongate shaft 14 about the rotational axis of the elongate shaft 14.

As discussed later herein, the distal end of the sleeve 40 may include one or more engagement features 44 configured to engage with the interior of the housing 56 of the bone screw assembly 50 through rotational movement of the sleeve 40 relative to the housing 56 of the bone screw assembly 50. For example, the engagement features 44 of the sleeve 40 may be tabs or protrusions extending in opposite directions from the sleeve 40. In some instances, the engagement features 44, e.g., tabs or protrusions, may extend generally perpendicular to the longitudinal axis of the sleeve 40.

The embodiment of FIG. 2A is similar to that of FIG. 2, with the exception of the engagement feature 44. As shown in FIG. 2A, in other instances, the engagement feature 44 of the sleeve 40 may include a threaded region on an exterior of the sleeve 40, such as discontinuous external threading positioned on opposing sides of the sleeve 40.

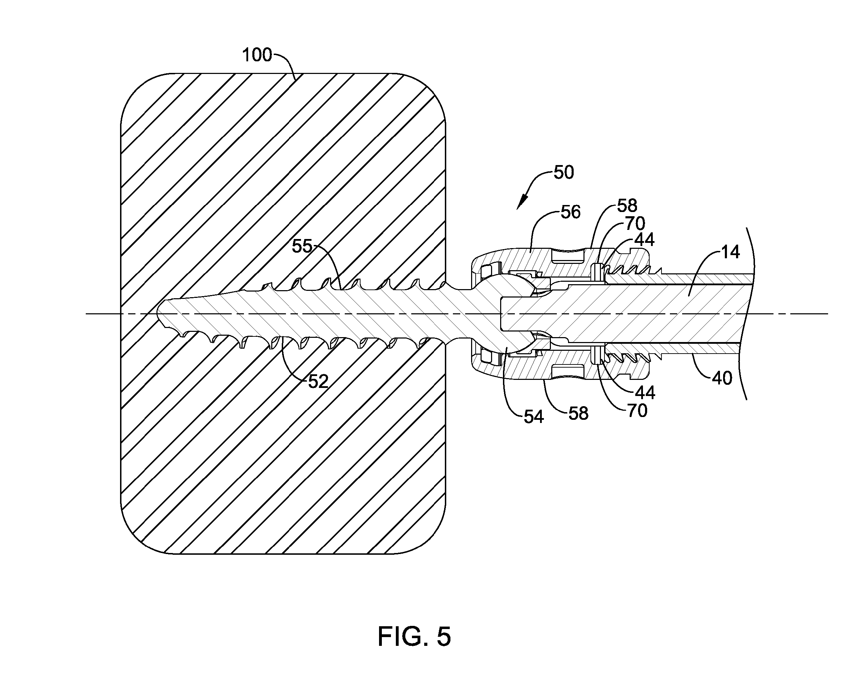

FIGS. 5-10 illustrate exemplary aspects of using the driver instrument 10 of FIG. 1 for installing a bone anchor assembly. As shown in FIG. 5, a bone anchor 50 may be installed in a bony structure 100 (e.g., a vertebra, sacrum, etc.) with the driver instrument 10. The bone anchor assembly 50 may include a threaded bone screw 52 having a head 54 and a threaded shank 55 extending distally from the head 54. The threaded shank 55 may be configured to be screwed into the bony structure 100. The bone anchor assembly 50 may also include a housing 56 coupled to the bone screw 52, such as rotatably and/or pivotably coupled to the bone screw 52.

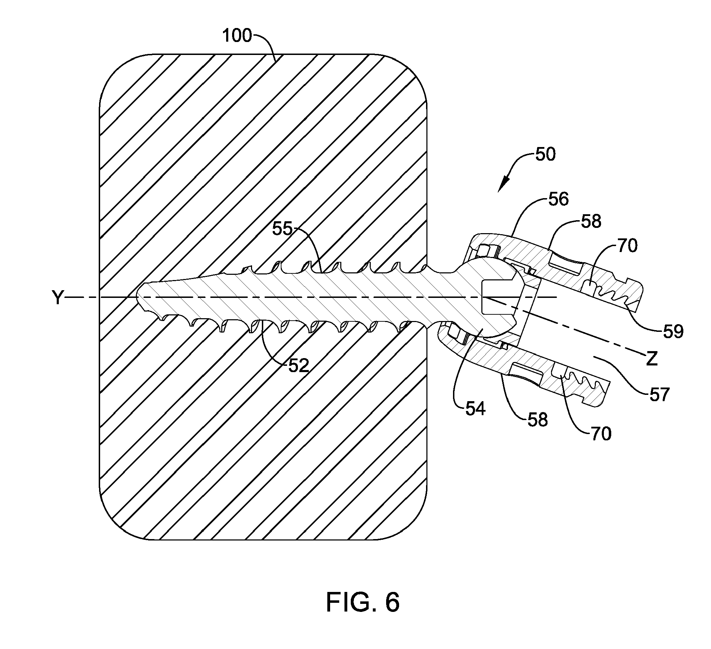

The housing 56 may be adapted to receive and retain an elongate stabilization member (e.g., a spinal rod, a flexible member, etc., not shown) therein for constructing a stabilization system. For example, the housing 56 may include a pair of legs 58 defining a pair of opposed channels 57, such as U-shaped channels, (see FIG. 6) therebetween at opposing sides of the housing 56, said channels adapted to receive an elongate stabilization member. A locking member, such as a set screw (not shown for clarity), may threadably engage a threaded axial opening 59 between the legs 58 to retain the elongate stabilization member in the channels 57.

In some embodiments, the housing 56 may be movably coupled to the typically spherical head 54 of bone screw 52 such that the housing 56 may be pivoted and/or rotated relative to the threaded shank 55 of the bone screw 52. For example, the bone anchor assembly 50 may be a polyaxial screw in which the housing 56 may be pivoted and rotated in a plurality of orientations relative to the bone screw 52. For example, the bone screw 52 may have a longitudinal rotational axis Y (shown in FIG. 6) and the housing 56 may have a longitudinal axis Z, wherein the longitudinal axis Z of the housing 56 may be angled at a plurality of angles relative to the longitudinal rotational axis Y by pivoting the housing 56 about the head 54 of the bone screw 52. Furthermore, in some instances the housing 56 may be rotated relative to the head 54 of the bone screw 52 about the longitudinal axis Z.

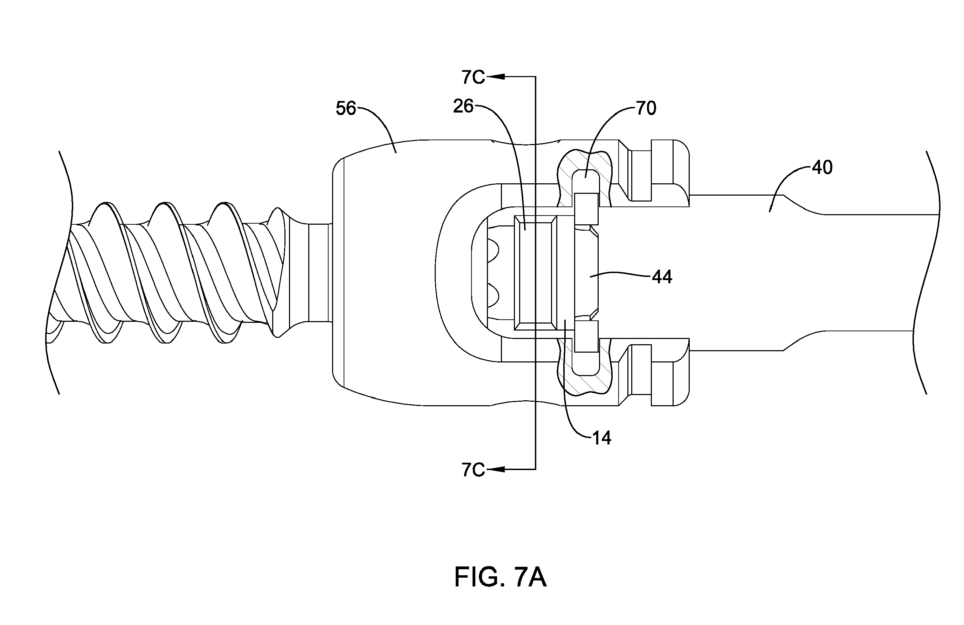

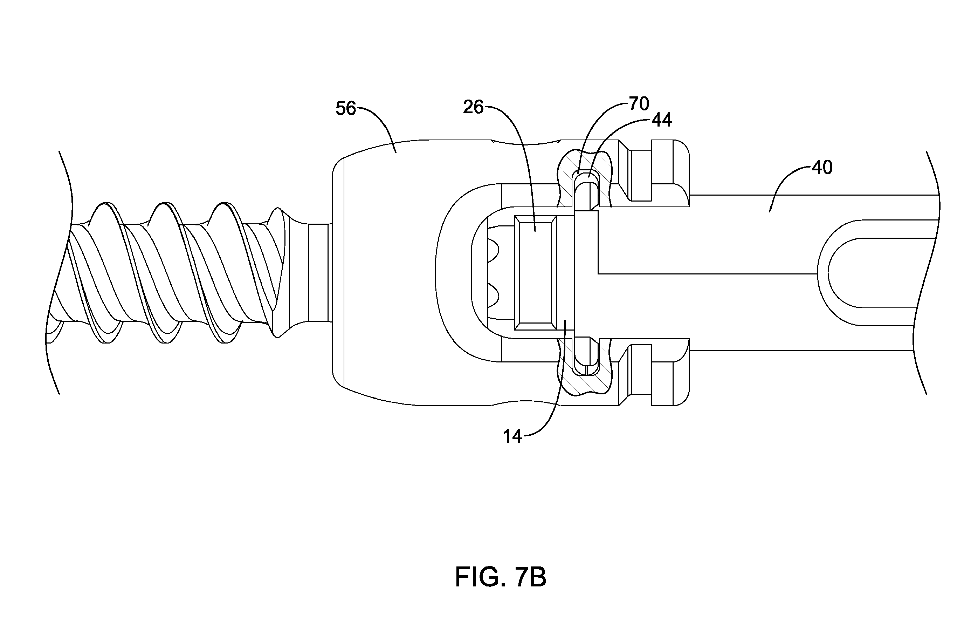

As will be seen in FIGS. 7A and 7B, the housing 56 may include one or more mating engagement features 70, in this example opposing thread reliefs (e.g., grooves, recesses) in legs 58 at the base of the internal threading, adapted to engage engagement features 44 of sleeve 40. In other instances, the mating engagement features 70 may be other grooves or recesses formed in the opposing legs 58. It will be appreciated that the engagement feature 44 and mating engagement feature 70 may be provided in a variety of forms, for example, in some embodiments the engagement feature 44 of the sleeve 40 may comprise a pair of opposed radial projections extending circumferentially at least partially around the sleeve and adapted to engage a mating engagement feature 70 of the housing 56, as also shown in FIGS. 7A and 7B, in which the projections providing the engagement feature 44 of sleeve 40 extend radially outward therefrom, said projections being sized and adapted to be slidingly inserted axially into the U-shaped channels 57 defined by legs 58 (FIG. 6) and subsequently rotated with the sleeve 44 about the longitudinal axis of the housing 56 to engage mating engagement features 70 of housing 56 in the form of circumferential thread reliefs or grooves in the inner surface of the legs 58 (FIG. 6) thereby preventing axial withdrawal of the sleeve 40 from the housing 56 and preventing the axial withdrawal of the driver engagement feature 20 from the driver socket 53 of the bone screw 52.

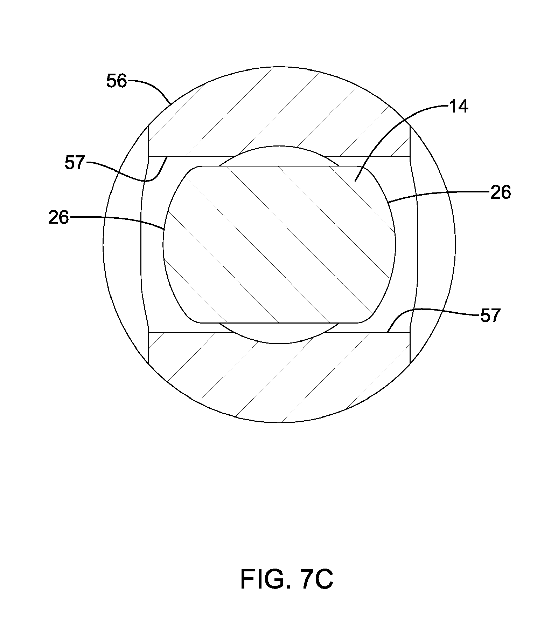

During subsequent rotation of the sleeve 40 and the engagement feature 44 relative to the housing 56 from the configuration of FIG. 7A to the configuration of FIG. 7B, turning of shaft 14 with the sleeve 40 is limited by the interaction of the lugs 26 within the longitudinal U-shaped channels 57 in the housing 56, said lugs 26 having preceded the engagement feature 44 into the longitudinal U-shaped channels 57. Lugs 26 of the shaft 14 and the engagement feature 44 of the sleeve 40 are adapted to at least partially enter channels 57, when aligned therewith as shown in FIG. 7C, for insertion into the housing 56 of the bone screw assembly 50 which allows the driver engagement feature 20 of the elongate shaft 14 to enter and mate with a complementary driver engagement feature (e.g., a driver socket 53) formed in a head 54 of a bone screw 52. Thus, the lugs 26 limit rotation of the shaft 14 relative to the housing 56 through interference with the edges of the legs 58 defining the channels 57. See, for example, FIG. 7C showing the relative position of the lugs 26 in the channels 57 on opposing sides of the housing 56 for limiting relative rotation between the housing 56 and the elongate shaft 14. Rotation of the sleeve 44 relative to the shaft 14 (shown in FIG. 7B), such as rotatably displaced by about 90 degrees, serves to couple the driver instrument 10 within the housing 56 of the bone screw assembly 50 thereby preventing withdrawal of the driver instrument 10 from the bone screw assembly 50 while maintaining longitudinally axial alignment between the bone screw 52 and the driver instrument 10.

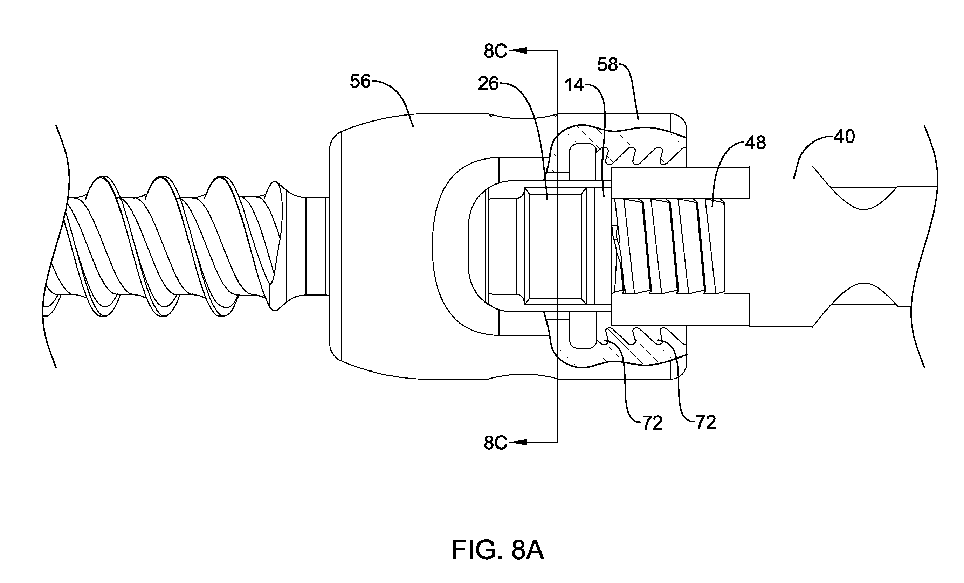

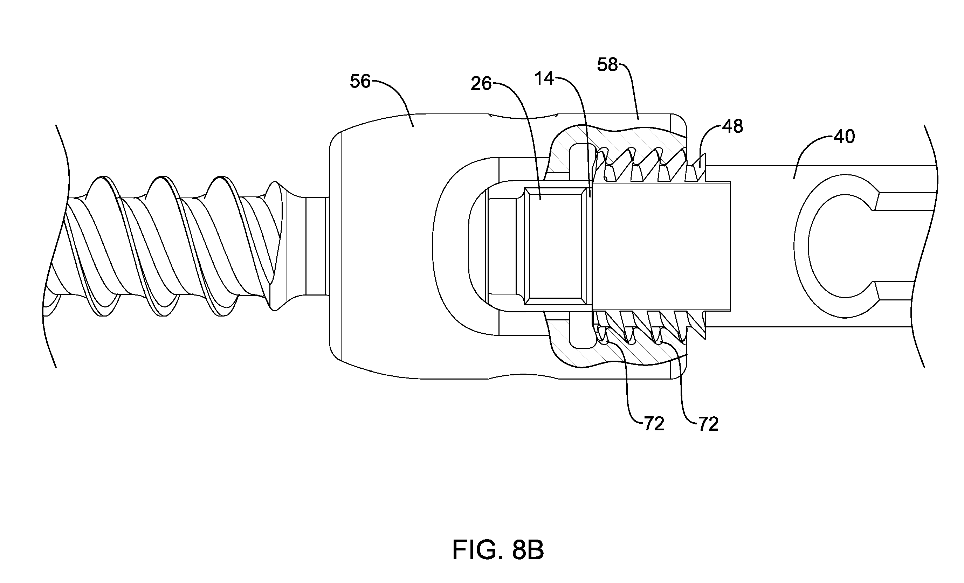

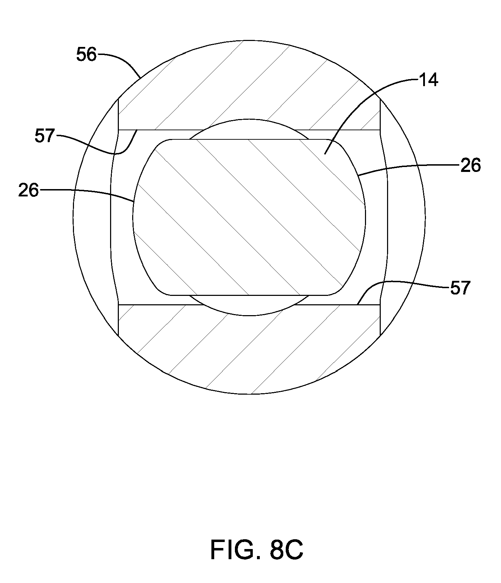

In alternate embodiments of the engagement feature 44 of sleeve 40 and mating engagement feature 70 of housing 56, shown in FIGS. 8A and 8B, the engagement feature 44 may be formed as a plurality of axially distributed projections 48, such as discontinuous external threading or otherwise resembling arc segments of male threads, and the mating engagement feature 70 may formed as internal threading 72 in the legs 58 of the housing 56, or otherwise a plurality of axially distributed thread-like grooves, resembling female threads, to accommodate variations in the degree to which the driver instrument 10 has been axially advanced relative to housing 56 prior to rotation of the sleeve 40 relative to housing 56. In some embodiments, the mating engagement feature 70 of housing 57 may be the threads adapted to receive a locking member (e.g., a set screw) mentioned previously. As before, the shaft 14 bearing lugs 26 precedes the sleeve 40 and the axially distributed projections 48 of the engagement feature 44 into longitudinal U-shaped channels 57 in housing 56 in a first configuration (Fig. 8A) whereupon the axially distributed projections 48 forming the engagement feature 44 may be laterally aligned with the adjacent grooves 72 of the mating engagement feature 70 prior to being rotated, typically about 90 degrees, to engage the housing 56 of the bone screw assembly 50. FIG. 8C illustrates the relative position of the lugs 26 in the channels 57 on opposing sides of the housing 56 for limiting relative rotation between the housing 56 and the elongate shaft 14. With the embodiment of FIGS. 8A-8C, rotation of the sleeve 40 relative to the housing 56 (and the shaft 14) through less than 180 degrees, for example about 90 degrees, permits multiple threads of the discontinuous threads to engage in the threading of the housing 56, resulting in secure engagement between the housing 56 and the sleeve 40.

Although the engagement feature 44 and mating engagement feature 70 have been characterized as projections and channels or grooves, respectively, including external threaded regions and internal threaded regions, respectively, it will be appreciated that the engagement feature 44 may comprise one or more channels or grooves and the mating engagement feature 70 may comprise one or more projections. Similarly, one of ordinary skill in the art will appreciate that functionally equivalent embodiments (not shown) may be formed in which the engagement feature 44 of the sleeve 40 is present on an inner surface of the sleeve 60 while the mating engagement feature 70 of the housing 56 may be present on an exterior surface thereof.

In certain embodiments, the projections and channels or grooves of the engagement feature 44 and mating engagement feature 70 may lie in a transverse plane substantially perpendicular to the axis of the housing 56 as illustrated in FIGS. 7A and 7B, while in certain other embodiments the projections and channels of the engagement feature 44 and mating engagement feature 70 may lie in planes which are tilted or angled relative to the axis of the housing 56 in the manner of mating screw threads of FIGS. 8A and 8B such that rotation of the sleeve 40 relative to the housing 56 during rotational engagement between the engagement feature 44 and mating engagement feature 70 tends to further insertion of the distal tip 22 into the driver socket 53.

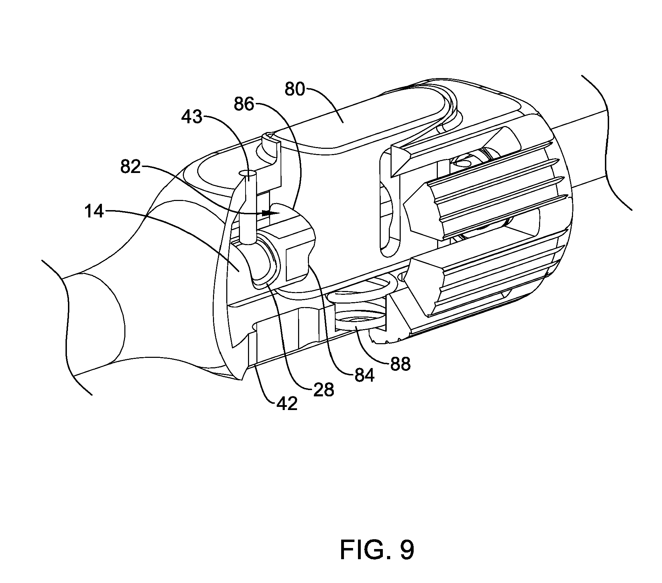

The sleeve handle 42 may also include an actuation mechanism including an actuator, such as a button 80, actuatable between a first position and a second position in which the elongate shaft 14 and the sleeve 40 are rotationally coupled and decoupled, respectively. In the first position, the button 80 or other actuator selectably engages elongate shaft 14 in a manner which prevents relative rotation between the elongate shaft 14 and the sleeve 40. In the second position, the button 80 or other actuator of the sleeve handle 42 selectably decouples from the elongate shaft 14 and allows relative rotation between the elongate shaft 14 and the sleeve 40 about the central longitudinal axis of the elongate shaft 14. Coupling/decoupling between the elongate shaft 14 and the sleeve 40 and/or sleeve handle 42 may be effected by any convenient means known in the art. An illustrative, non-limiting example, shown in FIG. 9, employs an aperture 82 through the button 80 which accommodates the elongate shaft 14 therethrough. The aperture 82 includes a narrow region 84 and a somewhat wider region 86, wherein the narrow region 84 is adapted to engage flat sides of a square portion of the elongate shaft 14 in the first position thereby preventing relative rotation and the somewhat wider region 84 is adapted to have sufficient clearance about the elongate shaft 14 to allow the elongate shaft 14 to freely rotate within the wider region 86. In other words, the width of the narrow region 84 may be less than the diameter of the elongate shaft 14, but greater than a distance between opposing side surfaces (e.g., flat surfaces) of the elongate shaft 14, while the width of the wider region 86 may be greater than the diameter of the elongate shaft 14 to permit rotation of the elongate shaft 14 therein. The sleeve handle 42 may also include a spring 88 which tends to bias the button 80 toward the first position. Applying pressure to the button 80 overcomes the bias provided by the spring 88 allowing the button 80 to move from the first position to the second position such that rotation of the sleeve handle 42 may rotate the sleeve 40 relative to the elongate shaft 14.

In addition, the actuation mechanism, such as the button 80 and/or the sleeve handle 42, may be adapted to limit rotation of the sleeve 40 relative to the elongate shaft 14. In certain embodiments, an interaction between the elongate shaft 14 and the aperture 82 of the button 80 suffices to limit rotation of the sleeve 40 relative to the elongate shaft 14 to increments of 90 degrees. In such embodiments, relative rotation of the shaft 14 and the sleeve 40 from a first configuration, in which engagement feature 44 may axially enter channels 57 of the housing 56 when the button is in the first position, to a second configuration allows rotation of engagement feature 44 into engagement with mating engagement feature 70 associated with the legs 58 of the housing 56, while the button 80 is in the second position. In the second configuration, engagement between engagement feature 44 and mating engagement feature 70 effectively couple the sleeve 40, elongate shaft 14, housing 56, and bone screw 52 into a single coaxial unit when the button 80 returns to the first position, such that the driver engagement feature 20 is seated in the driver socket 53 of the head 54 of the bone screw 52, said single coaxial unit being adapted to advance the bone screw 52 into a bony structure 100 when the elongate shaft 14 and sleeve 40 are turned by rotating one or both of the handle 12 and the sleeve handle 42.

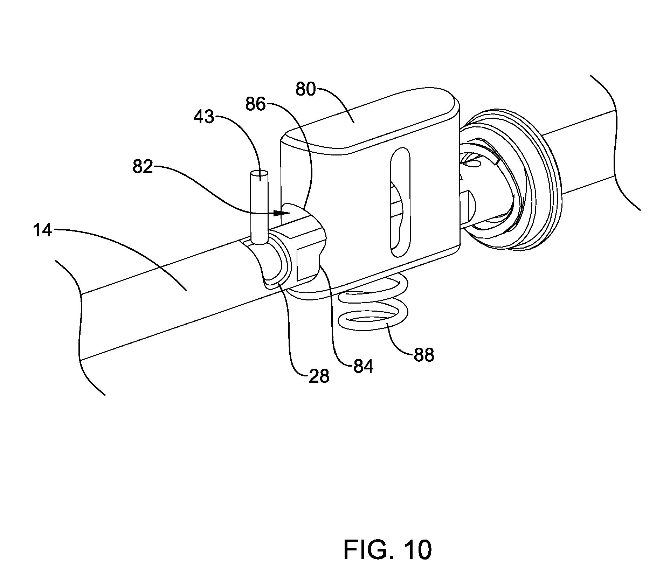

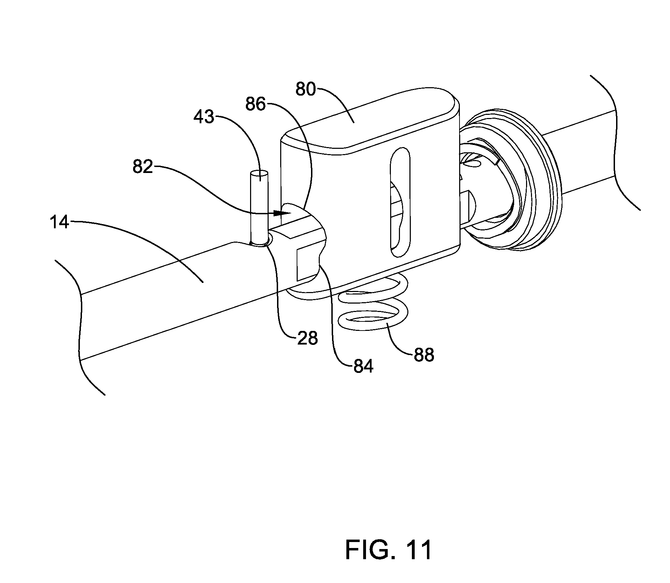

In some embodiments, rotation also may be limited by the inclusion of a pin 43 of the sleeve handle 42 which engages an arcuate groove 28 in the elongate shaft 14 as shown in FIG. 9. In other embodiments, a pin associated with the elongate shaft 14 may engage a feature on either the button 80, the sleeve handle 42 and/or the sleeve 40. FIG. 10 illustrates the embodiment of FIG. 9 with surrounding structures removed to improve clarity. As illustrated, the pin 43, anchored in the sleeve handle 42, rides in a groove 28 of the shaft 14 to limit rotation of the sleeve 40 relative to the shaft 14 to approximately 90 degrees as shown in FIG. 11. In yet other embodiments, the limited rotation feature may be associated with stops and/or detents provided on one or more of projections 46, projections 48, and grooves 72 of the engagement feature 44 and/or mating engagement feature 70, for example. Equivalent components adapted to limit rotationally couple/decouple the elongate shaft 14 and the sleeve 40 may be implemented by one of ordinary skill in the art.

The driver instrument 10 may be used to select and quickly engage a polyaxial bone anchor assembly 50 comprising a housing 56 and bone screw 52 from an assortment of such screws held in a surgical kit. In the alternative, a single polyaxial screw may be held between the fingers of an operator while the driver instrument 10 is engaged and coupled to the screw.

In use, the distal end 18 of the elongate shaft 14 may be inserted through the housing 56 and into the driver socket 53 in the head 54 of the bone screw 52 which positions the lugs 26 of the elongate shaft 14 in the channels 57 of the housing 56. The lugs 26 limit rotation of the elongate shaft 14 within the housing 56 while interactions among the elongate shaft 14, lugs 26, the driver engagement feature 20 and the driver socket 53 of the bone screw 52 coaxially align the elongate shaft 14, sleeve 40, housing 56, and bone screw 52. Engagement feature 44 of the sleeve 40 follows the lugs 26 into the channels 57 of the housing 56 and is initially positioned within the channels 57. In the illustrated embodiments, the distal end of the sleeve 40 may be inserted between the legs 58 of the housing 56; however in some embodiments the distal end of the sleeve 40 may surround the housing 56 if desired. When the button 80 of the sleeve handle 42 is depressed, the sleeve 40 is free to rotate relative to the elongate shaft 14 thereby allowing the engagement feature 44 of the sleeve 40 to rotate into engagement with the mating engagement feature 70 of the housing 56 while the lugs 26 of the elongate shaft 14 limit rotation of the housing 56. As noted herein, rotation of the engagement feature 44 of the sleeve 40 relative to the elongate shaft 14, and thus relative to the housing 56, may be limited to about 90 degrees by cooperating features of the elongate shaft 14 and the sleeve 40 or sleeve handle 42. Releasing the button 80 of the sleeve handle 42 couples the elongate shaft 14 relative to the sleeve 40 such that turning one of the handle 12, the elongate shaft 14, or the sleeve handle 42 turns both the housing 56 and the bone screw 52 allowing the driver instrument 10 to drive the bone screw 52 into a bony structure 100.

Once the bone screw 52 is properly seated in the bony structure 100, the button 80 may again be depressed allowing the sleeve 40 and thus the engagement feature 44 to counter-rotate to disengage the engagement feature 44 from the mating engagement feature 70 of the housing 56. Once the engagement feature 44 is repositioned within the channels 57 of the housing 56 thereby releasing the sleeve 40 and the elongate shaft 14 from the housing 56, the sleeve 40 and the elongate shaft 14 may be removed from the housing 56 and the bone screw 52.

Those skilled in the art will recognize that aspects of the present disclosure may be manifested in a variety of forms other than the specific embodiments described and contemplated herein. Accordingly, departures in form and detail may be made without departing from the present disclosure as described in the appended claims.

## Claims

1. A driver instrument adapted to engage and drive bone screw assemblies which include a bone screw and an associated housing, said driver instrument comprising:
an elongate shaft having a proximal end and a distal end, the distal end further comprising:
a driver engagement feature adapted to engage and rotationally drive a head of a bone screw and
one or more lugs protruding radially therefrom,
said one or more lugs being adapted to engage a housing of a bone screw assembly to limit axial rotation thereof relative to said elongate shaft; and
a sleeve having a proximal end, a distal end, and being adapted for coaxial rotation about a common longitudinal axis with the elongate shaft,
wherein the distal end of the sleeve includes an engagement feature adapted to rotationally engage a mating engagement feature of the housing of a bone screw assembly.

2. The driver instrument of claim 1, wherein the engagement feature of the sleeve includes one or more radial projections extending circumferentially at least partially around the sleeve and adapted to engage the mating engagement feature of the housing of the bone screw assembly.

3. The driver instrument of claim 2, wherein the mating engagement feature of the housing of the bone screw assembly is grooves of a threaded opening of the housing.

4. The driver instrument of claim 1, wherein the engagement feature of the sleeve includes one or more grooves extending circumferentially at least partially around the sleeve and adapted to engage the mating engagement feature of the housing of the bone screw assembly.

5. The driver instrument of claim 4, wherein the mating engagement feature of the housing of the bone screw assembly is threads of a threaded opening of the housing.

6. The driver instrument of any one of the preceding claims, wherein the proximal end of the sleeve includes a sleeve handle adapted to selectably couple to the elongate shaft to limit relative rotation therebetween about the common longitudinal axis.

7. The driver instrument of claim 6, wherein the sleeve handle adapted to selectably couple to the elongate shaft includes a button associated with the sleeve handle, said button having a first position in which the sleeve handle is rotationally coupled to the elongate shaft and a second position in which the sleeve handle and the elongate shaft are decoupled such that the sleeve handle may be rotated relative to the elongate shaft.

8. The driver instrument of any one of the preceding claims, wherein the elongate shaft includes a tapered distal tip adapted to facilitate insertion of the driver engagement feature into the head of the bone screw.

9. The driver instrument of any one of the preceding claims, wherein the proximal end of the elongate shaft further comprises a handle.

10. The driver instrument of claim 9, wherein the handle is removably coupled to the proximal end of the elongate shaft with a quick-connect feature.

11. The driver instrument of any one of the preceding claims, wherein the engagement feature of the sleeve lies in a plane transverse to the common longitudinal axis of the driver instrument.

12. The driver instrument of any one of the preceding claims, wherein the engagement feature of the sleeve lies in a plane substantially perpendicular to a longitudinal axis of the housing.

13. The driver instrument of any one of the preceding claims, wherein the engagement feature of the sleeve lies in one or more planes which are tilted relative to a longitudinal axis of the housing.

14. The driver instrument of any one of the preceding claims, wherein rotation of the engagement feature of the sleeve relative to the mating engagement feature of the housing tends to advance a distal tip of the elongate shaft into a driver socket of the bone screw assembly.

15. A method of engaging and a driver instrument with a bone screw assembly comprising:
inserting a distal tip of a driver instrument having an elongate shaft including a driver engagement feature into a driver socket of a bone screw of a bone screw assembly;
inserting one or more lugs of the elongate shaft into mating channels of a housing of the bone screw assembly, wherein inserting one or more lugs of the elongate shaft into mating channels of the housing of the bone screw assembly limits rotation of the housing relative to the elongate shaft;
inserting a sleeve coaxially disposed about the elongate shaft into the housing of the bone screw assembly with the sleeve in a first position, said sleeve including an engagement feature being adapted to rotationally engage a mating engagement feature of the housing of the bone screw assembly; and
rotating the sleeve relative to the elongate shaft and the housing of the bone screw assembly to a second position while positioned in the housing of the bone screw assembly, wherein upon relative rotation of the sleeve with respect to the elongate shaft, the engagement feature of the sleeve couples with the mating engagement feature of the housing of the bone screw assembly.
